# EUROPEAN PATENT APPLICATION

(11) **EP 3 246 445 A1**
(43) Date of publication of application: **22.11.2017**
(21) Application number: 17171705.1
(22) Date of filing: 18.05.2017
(51) Int. Cl.: D04H 1/728, A61K 9/70, A61K 31/202

(54) **LIPOXIN A4 DELIVERY SYSTEM BY A POROUS ELECTROSPUN POLYMERIC MEMBRANE**

(30) Priority: 19.05.2016 IT UA20163595
(71) Applicant: Universita' Degli Studi G. D Annunzio Chieti - Pescara, 66100 Chieti (IT)
(72) Inventor: CROCE, Fausto, 66100 Chieti (IT); ROMANO, Mario, 66100 Chieti (IT); TRUBIANI, Oriana, 66100 Chieti (IT); CIANCI, Eleonora, 66100 Chieti (IT); BRUNI, Pantaleone, 66100 Chieti (IT); DIOMEDE, Francesca, 66100 Chieti (IT); MESCHINI, Ida, 66100 Chieti (IT)
(74) Representative: Currado, Luisa

(57) **Abstract**

A delivery system for lipoxin of LXA₄, consisting of a polymeric porous matrix consisting of nanofibers of poly(ethylene oxide) (PEO) and poly(D,L-lactide) (PDLLA) obtained by electrospinning and it use for the treatment of periodontal diseases are disclosed.

## Description

### Background of the invention

The present invention refers to the field of nanotechnologies and more in particular to the sector of materials prepared by electrospinning, because it discloses a system for the delivery of lipoxin A₄ (LXA₄) by a porous polymeric matrix made of nanofibers. The present invention also refers to the pharmaceutical field because said delivery system is used for the treatment of several dental pathologies.

### State of the art

Electrospinning is a nanotechnology-based technique used to produce long polymer fibers of various materials. The technique provides applying an electric force, using a high strength electric field, to a viscous polymer solution that flows from the tip of a narrow metallic needle, to produce continuous polymer fibers, which are deposited onto a metallic collector to form a non-woven fibrous mat with micrometric thickness, the fibers have a diameter that ranges from a few tens of nanometers up to a few micrometers, depending on the polymer and solution properties (Bhardwaj N, Kundu SC. Electrospinning: a fascinating fiber fabrication technique, Biotechnology advances, 2010,28(3):325-47). The fibers network form a porous grid ideal for the release of active ingredients and for supporting genetic and cellular material (Boland ED, Wnek GE, Simpson DG, Pawlowski KJ, Bowlin GL, Tailoring tissue engineering scaffolds using electrostatic processing techniques: a study of poly (glycolic acid) electrospinning, Journal of Macromolecular Science, Part A. 2001;38(12):1231-43; Kim TG, Lee DS, Park TG. Controlled protein release from electrospun biodegradable fiber mesh composed of poly(epsilon-caprolactone) and poly(ethylene oxide). International journal of pharmaceutics. 2007;338(1-2):276-83). The active ingredient release can be tailored by modifying, for example, the polymer type, the concentration and the mixture, the possible e coating and the state of drugs included in the fibers (Zeng J, Xu X, Chen X, Liang Q, Bian X, Yang L, et al. Biodegradable electrospun fibers for drug delivery. Journal of controlled release: official journal of the Controlled Release Society. 2003;92(3):227-31; Jiang H, Hu Y, Li Y, Zhao P, Zhu K, Chen W. A facile technique to prepare biodegradable coaxial electrospun nanofibers for controlled release of bioactive agents. Journal of controlled release: official journal of the Controlled Release Society. 2005;108(2-3):237-43; Xu X, Yang L, Wang X, Chen X, Liang Q, Zeng J, et al. Ultrafine medicated fibers electrospun from W/O emulsions. Journal of controlled release: official journal of the Controlled Release Society. 2005;108(1):33-42; Zeng J, Aigner A, Czubayko F, Kissel T, Wendorff JH, Greiner A. Poly(vinyl alcohol) nanofibers by electrospinning as a protein delivery system and the retardation of enzyme release by additional polymer coatings. Biomacromolecules. 2005;6(3):1484-8. Epub 2005/05/10.)

It is known that membranes consisting of polymeric solid blends of poly(ethylene oxide) (PEO) and poly(D,L-lactide) (PDLLA), at different percent weight ratios, present variable degradation rates, in aqueous solution and at ambient temperature, depending on the PEO and PDLLA content. It is also known that the higher the PEO content in the solution, the faster is the degradation rate of the membrane (Kim TG, Lee DS, Park TG. Controlled protein release from electrospun biodegradable fiber mesh composed of poly(epsilon-caprolactone) and poly(ethylene oxide) International journal of pharmaceutics. 2007;338(1-2):276-83). Accordingly, depending on the nature and function of the encapsulated active ingredient, the relative content of PEO and PDLLA is selected, to meet the desired release profile of the active ingredient (Savva I, Odysseos AD, Evaggelou L, Marinica O, Vasile E, Vekas L, et al. Fabrication, characterization, and evaluation in drug release properties of magnetoactive poly(ethylene oxide)-poly(L-lactide) electrospun membranes. Biomacromolecules. 2013;14(12):4436-46).

It is known that periodontal diseases are widely associated with persistent inflammation, that can become chronic enhancing tissue destruction and bone resorption, contributing so to the failure and/or delay of tissue healing (Elemek E, Almas K. Peri-implantitis: etiology, diagnosis and treatment: an update. The New York state dental journal. 2014;80(1):26-32; Thomas MV, Puleo DA. Infection, inflammation, and bone regeneration: a paradoxical relationship. Journal of dental research. 2011;90(9):1052-61; Van Dyke TE, Serhan CN. Resolution of inflammation: a new paradigm for the pathogenesis of periodontal diseases. Journal of dental research. 2003;82(2):82-90; Van Dyke TE. The management of inflammation in periodontal disease. Journal of periodontology. 2008;79(8 Suppl):1601-8).

Lipoxin A₄ (LXA₄) is a metabolite of arachidonic acid, derived from the lipoxygenation, with potent anti-inflammatory properties, and able to regulate leucocyte trafficking and prevent necrosis and bone loss in a rabbit model of periodontitis (Serhan CN, Jain A, Marleau S, Clish C, Kantarci A, Behbehani B, et al. Reduced inflammation and tissue damage in transgenic rabbits overexpressing 15-lipoxygenase and endogenous anti-inflammatory lipid mediators. J Immunol. 2003;171(12):6856-65). Furthermore, LXA₄ stimulates proliferation, migration and wound healing properties of stem cells isolated from the periodontal ligament (hPDLSCs) (Cianci E, Recchiuti A, Trubiani O, Diomede F, Marchisio M, Miscia S, et al. Human Periodontal Stem Cells Release Specialized Proresolving Mediators and Carry Immunomodulatory and Prohealing Properties Regulated by Lipoxins. Stem cells translational medicine) and is as efficacious as steroids in the treatment of infantile eczema (Schottelius AJ, Giesen C, Asadullah K, Fierro IM, Colgan SP, Bauman J, et al. An aspirin-triggered lipoxin A4 stable analog displays a unique topical anti-inflammatory profile. J Immunol. 2002;169(12):7063-70).

The US patent n. US2007/0269555 describes nanofiber matrices, wherein inorganic metal oxide crystals are dispersed, obtained with the electrospinning method.

The Korean patent n. KR22060118937 describes an electrospinning procedure to obtain nano-fibrous mats.

The Korean patent n. KR20090120365 describes a poly-(N-vinylcarbazolo) non-woven fabric obtained with electrospinning.

### Technical problem

The therapeutic use of LXA₄, in its native form may be difficult. Indeed, is known that said active ingredient is rapidly metabolized into biologically-inactive compounds (Clish CB, Levy BD, Chiang N, Tai HH, Serhan CN. Oxidoreductases in lipoxin A4 metabolic inactivation: a novel role for 15-onoprostaglandin 13-reductase/leukotriene B4 12-hydroxydehydrogenase in inflammation. J Biol Chem. 2000 Aug 18;275(33):25372-80). For this reason several synthetic analogs with a higher half-life have been produced (Petasis NA, Akritopoulou-Zanze I, Fokin VV, Bernasconi G, Keled-jian R, Yang R, Uddin J, Nagulapalli KC, Serhan CN. Design, synthesis and bioactions of novel stable mimetics of lipoxins and aspirin-triggered lipoxins. Prostaglandins Leukot Essent Fatty Acids. 2005 Sep-Oct;73(3-4):301-21).

Said problem is crucial when conditions requires the local administration of lipoxin for tissue healing, regeneration and anti-inflammatory purposes, as for example in dental pathologies, surgical wounds, cutaneous and skin adnexa diseases.

In the pharmaceutical field are known cream formulation, for topical use, containing LXA₄, for the treatment of infantile eczema (Wu SH, Chen XQ, Liu B, Wu HJ, Dong L. Efficacy and safety of 15(R/S)-methyl-lipoxin A(4) in topical treatment of infantile eczema. Br J Dermatol. 2013 Jan;168(1):172-8).

Is evident the increasing interest for controlled releasing systems and medical scaffolds which can minimize the metabolic inactivation of LXA₄ and ensure its delivery in the site of action.

Surprisingly, the same inventors demonstrated that if LXA₄ is loaded in a membrane of poly(ethylene oxide) (PEO) and poly(D,L-lactide) (PDLLA), produced by electrospinning, can retained its biological properties, and thus inducing tissue healing and modulating periodontal ligament stem cells functions during periodontal pathologies.

Due to the rapid degradation and the great instability of LXA₄, the same inventors had to carefully calibrate the process and the system parameters to obtain a final product with the desired characteristics. The induced modification and calibrations, are not of the laboratory routine but they were specifically selected to solve the technical problem.

Indeed, in the present invention it was demonstrated that LXA₄ is effectively incorporated within the membrane and released when necessary, in the presence of an aqueous environment. The eluted fully retains its capability to induce cellular proliferation, reduce local inflammation and promote tissue healing in particular of cells deriving from the periodontal ligament.

### Object of the Invention

With reference to the attached claims are objects of the present invention:
A delivery system comprising a porous membrane of poly(ethylene oxide) (PEO) and poly(D,L-lactide) (PDLLA) and LXA₄ wherein
the membrane consisting of fibers of poly(ethylene oxide) (PEO) and poly(D,L-lactide) (PDLLA) with random distribution and having a diameter between 0,8 and 1,2 µm nanometer, in the membrane the average pore size is between 5 and 15 µm, the glass-transition temperature (Tg) of the membrane is between 45° and 50°C, the membrane shows an melting peak between 59° and 64 °C;

An electrospinning method for the preparation of the above delivery system comprising the steps of:
a) preparation of a polymer solution;
b) loading the polymeric solution prepared in a) into a glass syringe with a metallic needle;
c) application of an high voltage between the needle and a collecting metallic plate (collector);
d) spilling from the needle the polymer solution at a constant speed, with the formation of polymeric fibers which are deposited onto the collecting metallic plate (collector)to form a membrane;

Characterised in that in step a), to prepare the polymer solution, poly(ethylene oxide) (PEO) and poly(D,L-lactide) (PDLLA) are previously individually dissolved in N,N- dimethylformamide (DMF), and then mixed together and with LXA₄, the concentration of poly(ethylene oxide) (PEO) is comprised between 1% and 6% wt, the concentration of poly(D,L-lactide) (PDLLA) in the mixture is between 2% and 12% wt; wherein the percentage by weight is expressed in relation to the total weight of N,N- dimethylformamide (DMF);
in step c) the applied voltage is between 9 and 20 kv;
in step d) constant speed means that the solution flows at between 10 and 20 µl/min, the metallic needle tip is placed between 10 and 30 cm from collecting metallic plate (collector).

A further object of the present invention is a delivery system comprising a porous membrane of a poly(ethylene oxide) (PEO) and poly(D,L-lactide) (PDLLA) and LXA₄, wherein the membrane consisting of random oriented fibers of poly(ethylene oxide) (PEO) and poly (D,L-lactide) (PDLLA), fibers having a diameter between 0,8 and 1,2 µm and an average pores size between 5 and 15 µm, the membrane having a glass-transition temperature (Tg) between 45 and 50°C, having a melting peak between 59 and 64°C, obtained by an electrospinning process comprising the steps of:
a) preparation of a polymer solution;
b) loading the polymeric solution prepared in a) into a glass syringe with a metallic needle;
c) application of an high voltage;
d) spilling from the needle the polymer solution at a constant speed, with the formation of polymeric fibers which are deposited onto the collecting metallic plate (collector)to form a membrane;
characterised in that in step a), to prepare the polymer solution, poly(ethylene oxide) (PEO) and poly(D,L-lactide) (PDLLA) are previously individually dissolved in N,N- dimethylformamide (DMF), and then mixed together and with LXA₄, the concentration of poly(ethylene oxide) (PEO) is comprised between 1% and 6% wt, the concentration of poly(D,L-lactide) (PDLLA) in the mixture is between 2% and 12% wt; wherein the percentage by weight is expressed in relation to the total weight of N,N- dimethylformamide (DMF);
in step c) the applied voltage is between 9 and 20 kv;
in step d) constant speed means that the solution flows at between 10 and 20 µl/min, the metallic needle tip is placed between 10 and 30 cm from collecting metallic plate (collector).

A further object of the present invention is the above delivery system for use as a medicament.

A further object of the present invention is the above delivery system for the treatment of periodontal disease.

Additional characteristic of the present inventions will be evident from the following detailed description, with reference to the experimental data presented and the attached drawings.

### Brief description of the drawings

Figure 1 shows the weight-loss profile of PEO/PDLLA membranes maintained in buffer solution.
Figure 2 shows the thermogram of the pristine PEO/PDLLA membrane without active ingredient.
Figure 3 shows the viability of hPDLSCs cultured for 7 days onto PEO/PDLLA membranes compared to hPDLSCs grown on normal culture plate. Results are expressed as mean ± SEM from five independent experiments with quintuplicates.
Figure 4 shows the time course of LXA₄ release from PEO/PDLLA membrane maintained in PBS from 1 to 96 h. Results are expressed as percentage of total LXA₄. Data are mean ± SEM of 3 different LXA₄-loaded membrane preparations.
Figure 5 shows the percentage of LXA₄ isomerization (tLXA₄) on the total LXA₄ eluted from the membranes. Results are expressed as mean ± SEM of 3 different LXA₄-loaded membrane preparations.
Figure 6 shows the biological activity assay of hPDLSCs incubated for 24 or 48 h with LXA₄ (0.1-1 nM), either eluted from PEO/PDLLA membranes after 18 h incubation with PBS, or in its native form. hPDLSCs kept with basal medium or empty membrane mats were used as controls. Results are expressed as mean ± SEM of n = 4.
Figure 7 describes the biological activity assay of hPDLSCs incubated for 24 or 48 h with LXA₄ (0.1-1 nM), either eluted from PEO/PDLLA membranes after 72h incubation with PBS, or in its native form. hPDLSCs kept with basal medium or empty membrane mats were used as controls. Results are expressed as mean ± SEM of n = 4.

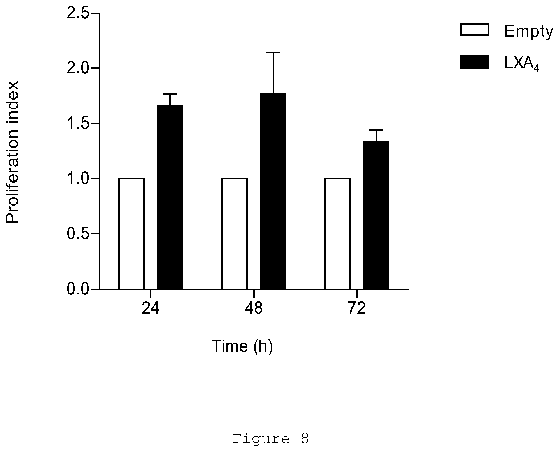

Figure 8 shows the effects of LXA₄ eluted from PEO/PDLLA membrane on hPDLSCs cultured in direct contact with 1 cm² sections of PEO/PDLLA membrane, empty or leaded with LXA₄, - for 24, 48 and 72 h. Results are expressed as mean ± SEM (n = 4).

### Detailed description of the invention

The object of the present invention is a delivery system comprising a porous membrane of poly(ethylene oxide) (PEO) and poly(D,L-lactide) (PDLLA) and LXA₄ wherein:
the membrane consisting of random oriented fibers of poly(ethylene oxide) (PEO) and poly(D,L-lactide) (PDLLA) having a diameter between 0.8 and 1,2 µm, in the membrane the average pores size is between 5 and 15 µm, the membrane having a glass-transition temperature (Tg) between 45° and 50°C and the membrane showing a melting peak between 59° and 64 °C.

Preferably, the fibers of poly(ethylene oxide) (PEO) and poly(D,L-lactide) (PDLLA) have a diameter of 1 µm.

Preferably, in the membrane the average pores size is 15 µm.

Preferably, the membrane has a glass-transition temperature (Tg) of 47°C.

Preferably, the membrane has a melting peak of 60°C.

Preferably, the thickness of the membrane is between 15-40 µm, more preferably between 20 and 25 µm and even more preferably of 20 µm.

The above delivery system is prepared by a electrospinning method comprising the following steps:
a) preparation of a polymer solution;
b) loading the polymeric solution prepared in a) into a glass syringe with a metallic needle;
c) application of an high voltage;
d) spilling from the needle the polymer solution at a constant speed, with the formation of polymeric fibers which are deposited onto the collecting metallic plate (collector)to form a membrane;
characterised in that in step a), to prepare the polymer solution, poly(ethylene oxide) (PEO) and poly(D,L-lactide) (PDLLA) are previously individually dissolved in N,N- dimethylformamide (DMF), and then mixed together and with LXA₄, the concentration of poly(ethylene oxide) (PEO) is comprised between 1% and 6% wt, the concentration of poly(D,L-lactide) (PDLLA) in the mixture is between 2% and 12% wt; wherein the percentage by weight is expressed in relation to the total weight of N,N- dimethylformamide (DMF);
in step c) the applied voltage is between 9 and 20 kv;
in step d) constant speed means that the solution flows at between 10 and 20 µl/min, the metallic needle tip is placed between 10 and 30 cm from collecting metallic plate (collector).

Preferably, in step a) the concentration of poly(ethylene oxide) (PEO) is 3% of the total N,N- dimethylformamide (DMF) weight.

Preferably, in step a) the concentration of poly(D,L-lactide) (PDLLA) in the mixture is 6% of the total N,N- dimethylformamide (DMF) weight.

Preferably, in step c) the applied voltage is between 10 and 12 kV.

Preferably, in step d) the needle tip is placed at a distance of 12 cm from the collecting metallic plate (collector).

Preferably, in step d) the solution flows at a rate between 13-15 µl/min.

Preferably, the collecting metallic plate (collector) is of aluminum.

A further object of the present invention is a delivery system comprising a porous membrane of a poly(ethylene oxide) (PEO) and poly(D,L-lactide) (PDLLA) and LXA₄, wherein the membrane consisting of randomly-oriented fibers of poly(ethylene oxide) (PEO) and poly(D,L-lactide) (PDLLA) fibers and having a diameter between 0,8 and 1,2 µm and an average pores size between 5 and 15 µm, the membrane having a glass-transition temperature (Tg) between 45 and 50°C, having a melting peak between 59 and 64°C, obtained by an electrospinning process comprising the steps of:
a) preparation of a polymer solution;
b) loading the polymeric solution prepared in a) into a glass syringe with a metallic needle;
c) application of an high voltage;
d) spilling from the needle the polymer solution at a constant speed, with the formation of polymeric fibers which are deposited onto the collecting metallic plate (collector)to form a membrane;
wherein in step a), to prepare the polymer solution, poly(ethylene oxide) (PEO) and poly(D,L-lactide) (PDLLA) are previously individually dissolved in N,N- dimethylformamide (DMF), and then mixed together and with LXA₄, the concentration of poly(ethylene oxide) (PEO) is comprised between 1% and 6% wt, the concentration of poly(D,L-lactide) (PDLLA) in the mixture is between 2% and 12% wt; wherein the percentage by weight is expressed in relation to the total weight of N,N- dimethylformamide (DMF); and in step c) the applied voltage is between 9 and 20 kv;
in step d) constant speed means that the solution flows at between 10 and 20 µl/min, the metallic needle tip is placed between 10 and 30 cm from collecting metallic plate (collector).

Preferably, the fibers of poly(ethylene oxide) (PEO) and poly(D,L-lactide) (PDLLA) have a diameter of 1µ.

Preferably, in the membrane the average pore size is 15µm.

Preferably, in the membrane the glass-transition temperature (Tg) is 47°C.

Preferably, the membrane have a melting peak at 60°C.

Preferably, the membrane have a thickness between 15-40 µm, more preferably between 20 and 25 µm, even more preferably of 20 µm.

Preferably, in the step a) the concentration of poly(ethylene oxide) (PEO) is 3%wt of the total N,N- dimethylformamide (DMF) weight.

Preferably, the concentration of poly(D,L-lactide) (PDLLA) in the mixture is 6%wt of the total N,N- dimethylformamide (DMF) weight.

Preferably, the voltage is between 10and 12 kV.

Preferably, the solution flows at a rate between 13 and 15 µl/min.

Preferably, the needle tip is placed 12 cm above the collecting metallic plate (collector).

Preferably, the collecting metallic plate (collector) is of aluminum.

In a preferred embodiment of the present invention, the delivery system comprising a porous membrane of poly(ethylene oxide) (PEO) and poly(D,L-lactide) (PDLLA), and lipoxine, wherein the membrane consisting of randomly oriented fibers of a poly(ethylene oxide) (PEO) and poly(D,L-lactide) (PDLLA) and having a diameter of 1µm; an average pore size of 15 µm; glass-transition temperature (Tg) at 47°C, and a melting peak at 60°C, obtained by dissolving PEO (Sigma-Aldrich, Milan) in 2.5 mL of N,N- dimethylformamide (DMF) at 40 °C under magnetic stirring for 24 h. PDLLA (Sigma-Aldrich) was separately dissolved in 1.5 mL of DMF at room temperature under magnetic stirring for 12 h. The two solutions were then mixed and stirred at room temperature for another hour. The concentrations of PEO and PDLLA in the DMF solution were respectively 4 % and 8 % of the total N,N-dimethylformamide (DMF) weight. Ten microliters of LXA₄ were added under continuous stirring and after 1 h incubation, the solution was transferred to a 5 mL glass syringe fitted with a metallic needle. The membrane was electrospun at 10-12 kV with solution flow rates in the range of 13-15 µL/min. The needle tip (anode) was placed above a collecting metallic plate (collector) of aluminum acted as static collector (cathode). The obtained mat dried under vacuum to remove any residual trace of solvent and stored at + 4 °C.

Object of the present invention is said delivery system for use as a medicament.

A further object of the present invention is said delivery system for the treatment of periodontal disease.

Within the meaning of the present invention periodontal disease means pathologies of periodontium, i.e. the set of structures supporting the tooth, comprising the alveolar bone, the periodontal ligament, the root cement and gum, in particular inflammatory conditions such as such as gingivitis, or periodontitis or paradontitis.

### Examples

### Material and methods

The membrane characterization performed using scanning electro-microscopy, images were taken with a Zeiss Evo50 Scanning Electron Microscope (Zeiss, Jena, Germany). Before analysis, the samples were covered by a thin layer of sputtered gold, using an Emitech K550 (Emitech Ltd., Ashford, UK) apparatus.

The kinetics of the PEO dissolution from the samples of pristine PEO/PDLLA membranes, is evaluated by recording the weight loss of the samples, after immersion in pure bi-distilled water for varying time. The samples are weighted using an analytical balance (Precisa XT 220A). After each immersion, the samples are dried under vacuum at room temperature before weight determination.

The differential scanning calorimetry analysis was carried out with a Mettler Toledo Differential Scanning Calorimeter, calibrated against an indium standard, and driven by the STAR Excellence software. An empty aluminum pan was used as reference material. The thermal protocol applied to the samples, typically 15-18 mg of fibrous mat, consisted in cycles under nitrogen atmosphere (flow rate 50 mL/min) at 10 °C/min. The samples, quenched at -80 °C, were t heated from -80 °C to 180 °C with an isothermal rest at 180 °C for 1 minute, then cooled again at -80 °C with an isothermal rest at -80 °C for 5 minutes. Finally, the samples were heated at 10 °C/min to 180 °C. The glass-transition temperature (Tg) and the melting temperature (Tm) were recorded in the second cycle.

The release of LXA₄ from the membrane was evaluated using phosphate-buffered saline solution (PBS) (pH 7.4, at 37°C). Pieces (3 cm x 2.5 cm, ∼33 mg, sample) of membranes, empty or LXA₄-loaded, were placed in 100 mm dishes containing PBS (8 mL) and maintained at 37 °C, 5% CO₂ and 95% humidity for 1, 3, 18, 48, 72 and 96 h. LXA₄ release was assessed by HPLC analysis after solid phase extraction. Following incubation with PBS, membranes were diluted with 2 volumes of cold MeOH and stored at -80°C until extraction. All samples were extracted using C-18 cartridges (Bond Elut Varian, Agilent Technologies, Milan) as previously described (Romano M, Luciotti G, Gangemi S, Marinucci F, Prontera C, D'Urbano E, et al. Urinary excretion of lipoxin A(4) and related compounds: development of new extraction techniques for lipoxins. Laboratory investigation; a journal of technical methods and pathology. 2002;82(9):1253-4). Before extraction, PGB₂ (50 ng/sample) was added to each sample as internal standard to calculate extraction recovery. Samples were then taken to dryness using a rotary evaporator, suspended in 10 mL of deionized (dd) H₂O containing MeOH, acidified to pH 3.5 and rapidly loaded onto C-18 columns previously equilibrated with MeOH and (dd) H₂O. After sample loading, columns were washed with (dd) H₂O to restore a neutral pH. Samples were eluted with 7 mL of methyl formate and taken to dryness using a Speedvac apparatus. The dried samples were then suspended in MeOH (100 µl) and injected into an HPLC apparatus (Agilent 1100) equipped reverse phase-HPLC with a Hypersil C-18 column (300 mm x 4 mm x 5 µm, Waters) and a photodiode array detector (Waters) for online UV spectroscopy. LXA₄ was identified by UV spectroscopy and considering the chromatographic retention time.

Bioptic samples of periodontal ligament were obtained from volunteers aged 20-35 years, exempt from systemic or oral disease, after undesigning the informed consent deed. The study was approved by the Medical Ethic Committee of the "G. D'Annunzio" University, Chieti, Italy (n°266/17.04.14). The periodontal tissue obtained by bioptic samples, grinded and washed several times with PBS (Lonza, Basel, Switzerland) was cultured using TheraPEAK™MSCGM-CD™ BulletKit serum free (MSCGM-CD) medium for the growth of human mesamchimal steam cells (MSCGM-CD) (Lonza), to minimize the exposure to substances of non-human origin. Human periodontal ligament stem cells (hPDLSCs) were obtained and cultured as reported in Trubiani O, Piattelli A, Gatta V, Marchisio M, Diomede F, D'Aurora M, et al. Assessment of an efficient xeno-free culture system of human periodontal ligament stem cells. Tissue engineering Part C, Methods. 2015;21(1):52-64.

Cells migrated from the explants and on day 7, 80-90% confluent adherent cells, were collected using 0.1% trypsin and seeded onto tissue culture polystyrene flasks at 5 x 10³ cells/cm², maintained at 37°C in a humidified atmosphere of 5% (v/v) CO₂. Primary cultures of hPDLSCs mainly consisted of colonies of bipolar fibroblast-like cells, which, after sub-cultivation, proliferated with a 48 h doubling time. Cell suspensions (2 x 10³ cells/well) were seeded on PEO/PDLLA membranes for different times or cultured in the absence of bio-membranes as the control. The interaction of cells with PEO/PDLLA membranes was monitored by SEM. Briefly, hPDLSCs (3 x 10³ cells/well) were suspended with MSCBM-CD and seeded onto PEO/PDLLA membranes for varying time. The samples were fixed for 4 h at 4°C with 4% glutaraldehyde in 0.05 M phosphate buffer (pH 7.4), dehydrated with increasing ethanol concentrations and dried. The samples were mounted on supports made by alluminium matrix coated with gold with an Emitech K550 (Emitech Ltd.) sputter-coater. Imaging was performed with a SEM EVO 50, ZEISS. ,). In all the experiments, the controls were hPDLSCs seeded without membranes.

Viability of hPDLSCs cultured on PEO/PDLLA membranes containing LXA₄ was evaluated by MTT. The cells (2 x 10³ cells/well) were seeded onto bio-membranes in a 96-well culture plate with MSCBM-CD medium and incubated for 24, 48 and 72 h and 1 week. After incubation, cells were washed with PBS, and the medium was replaced with 200 µL MTT in MSCBM-CD for 2 h. The optical density was read at 570 nm, using a ND-1000 NanoDrop1 Spectrophotometer (NanoDrop Technologies, Rockland, DE).

For the proliferation assay with LXA₄ released from PEO/PDLLA membrane, hPDLSCs (2 x 10³ cells/well) were seeded in 96-well microplates with complete medium overnight at 37 °C and 5%CO₂. The following day, cells were treated with: a) 0.1 and 1 nM LXA₄ released from LXA₄-loaded membranes after 18 h and 72 h incubation with PBS; b) commercial LXA₄ (Calbiochem, San Diego, CA, USA) at 0.1 and 1 nM concentration; c) PBS eluates of empty membranes; d) vehicle (0.01% ethanol). Cells were enumerated after 24 and 48 h using trypan blue dye as viability marker. Data were expressed as proliferation index, calculated as the ratio between values from cells exposed to membrane-eluted LXA₄ and from cells exposed to vehicle only (ethanol) of LXA₄. Each condition was tested in triplicate and experiments were repeated 3 times using hPDLSCs from different donors.

For proliferation assay with LXA₄-loaded PEO/PDLLA membranes, hPDLSCs (15 x 10⁴ cells/well) were seeded in 6-well plates with complete medium overnight at 37°C and 5 % CO₂. After 24 h, a small piece of 1 cm² surface and around 2mg weight, cut from LXA₄-loaded membranes, was added into each well. Cellular proliferation was evaluated after 24, 48 and 72 h using manual counting of trypan blue-excluding cells. Each condition was tested at least in triplicate and experiments were repeated 2 times.

The statistical significance for cell growth and cytotoxicity data was assessed with multiple comparison with Anova and Tukey tests using the Sigma Plot 9.0 software (Systat Software, Inc., Point Richmond, CA, USA).

### Example-1: membrane preparation

PEO (Sigma-Aldrich, Milan) was dissolved in 2.5 mL of N,N-dimethylformamide (DMF) at 40 °C under magnetic stirring for 24 h, PDLLA (Sigma-Aldrich) was separately dissolved in 1.5 mL of DMF at room temperature under magnetic stirring for 12 h. The two solutions were then mixed and stirred at room temperature for another hour. The concentrations of PEO and PDLLA in the DMF solution were 4 % and 8 % weight, compared to the total weight of of N,N-dimethylformamide (DMF), respectively. Ten microliters of LXA₄ were added under continuous stirring. and after 1 h incubation, the solution was transferred to a 5 mL glass syringe fitted with a metallic needle and set in the electrospinning apparatus. The flow of the polymer solution, from the syringe into the needle (5 cm length and 1.5 mm inner diameter), was controlled by a programmable syringe pump (KD Scientific). Membranes were electrospun at 10-12 kV with solution flow rates in the range of 13-15 pL/min. The needle tip (anode) was placed at 12 cm above a 10 x 10 cm² metallic plate, covered with aluminum foil that acted as static collector (cathode). The obtained mats were dried under rotary pump vacuum to remove any residual trace of solvent and stored at + 4 °C.

### Example-2: morphologic characterization

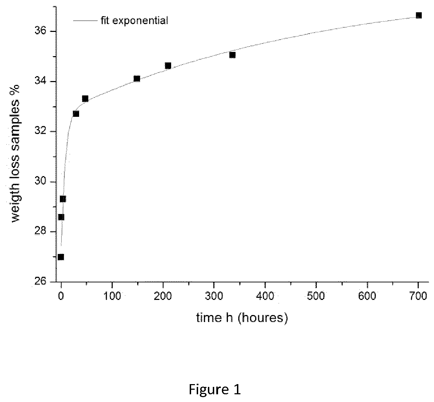

The membranes were prepared as indicated in the example 1. Average diameter and uniformity of the membrane fibers were evaluated by SEM (Fig. 1B-C). The dried electrospun mesh showed good mechanical stability that enabled easy handling. The grid showed good mechanical stability allowing easy handling. Images taken by electronic microscopy (not shown) of membranes not lipoxin-loaded, showed random but homogeneous distribution of the fibers, with regular texture and smooth surface. The average diameter of the nanofibers was around 1 µm. SEM analysis also revealed that fibers form a high porosity mat, with pores of around 15µm. The fibers revealed a compact structure, without any intrinsic porosity. After prolonged immersion of the membranes in a PBS buffer solution (0.1 M, pH 7.4), the fibers showed significant morphological changes and evident signs of deterioration. In particular, the water-soluble component, PEO, completely dissolved, leaving thinner and irregular fibers formed by the PDLLA component, which has a very low water solubility. In these extreme conditions the membranes, even if apparently retained their mechanical integrity, with a pore structure similar to that of the pristine mats, became less elastic and more fragile.

### Example-3: Weight loss test

The morphological changes of the membranes maintained in buffer solution were confirmed by monitoring membrane weight loss in bi-distilled water for different times. Figure 1 shows the typical weight-loss profile of PEO/PDLLA membrane over time. The sample undergoes a rapid weight loss of approximately 28% in the first hour of contact with water and an additional 6% was lost in a week. This was due to the release of PEO (36 %wt). The steady state weight loss was reached after approximately one month and the final loss in weight was equal to the PEO content of the samples.

### Example-4: Differential scanning calorimetry (DCS)

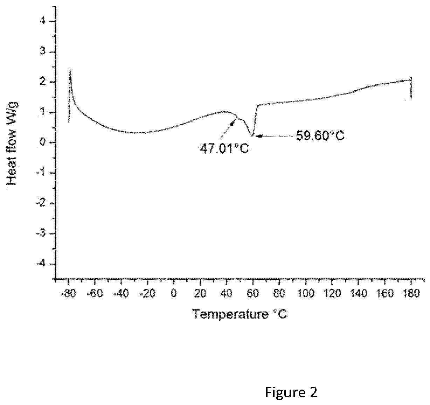

The DSC trace showed a endothermic peak at around 60 °C, as showed in Figure 2, corresponding to the melting of the crystalline fraction of the PEO component of the membranes, (as reported in Neto CG, Dantas TN, Fonseca JL, Pereira MR. Permeability studies in chitosan membranes. Effects of crosslinking and poly(ethylene oxide) addition. Carbohydrate research. 2005;340(17):2630-6; Caykara T, Demirci S, Eroglu MS, Güven O. Poly (ethylene oxide) and its blends with sodium alginate. Polymer. 2005;46(24):10750-7). Moreover, it is well known that the PDLLA has an amorphous structure (Kaitian X, Kozluca A, Denkbas E, Piskin E. Poly (D, L-lactic acid) homopolymers: Synthesis and characterization. Turkish Journal of Chemistry. 1996;20:43-53) with a glass-transition temperature (Tg), beteween 20 °C and 57 °C, depending on the average molecular weight, the dispersion index and the purity of the samples. Figure 2 shows a Tg at approximately 47 °C, which is consistent with the average molecular weight, of PDLLA (Mw 72.000), taking into account that PEO could reduce PDLLA chains mobility, which in turns increases the Tg.

### Example-5: hPDLSCs biocompatibility for PEO/PDLLA membranes

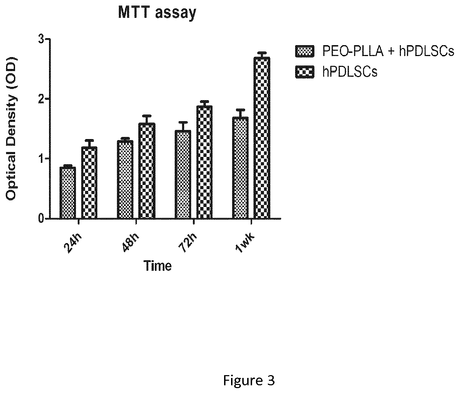

Cytocompatibility of PEO/PDLLA membranes, in particular hPDLSCs attachment and growth on the membrane, using SEM and MTT assays, respectively. Seven days after seeding, scanning electron microscopy at high magnification (500 x) showed that hPDLSCs adhered and spread within the membrane, displaying a homogeneous fibroblast-like morphology, with a stellate shape, similar to that of hPDLSCs grown on culture dishes. hPDLSCs produced filopodia and long active cytoplasmic processes that enabled the anchorage to the PEO/PDLLA membranes. hPDLSCs cultured on membranes displayed bridge structures, indices of shape changes to adapt with the seeding surface. The proliferation rate and viability of hPDLSCs cultured on the PEO/PDLLA membranes were assessed by the MTT assay at 1 to 7 days of culture. As shown in figure 3, although cell growth was slightly reduced by the presence of the membranes, particularly after 1 week, these differences did not reach statistical significance. Thus , these results indicate that the PEO/PDLLA membranes have high biocompatibility.

### Example-6: Release and biological activity of LXA₄ from PEO/PDLLA membranes

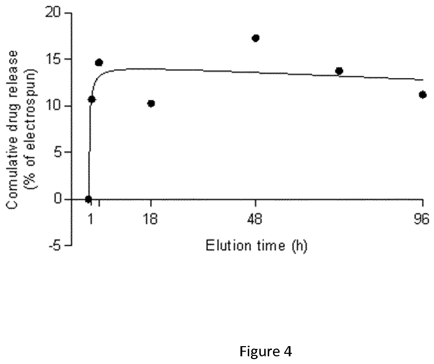

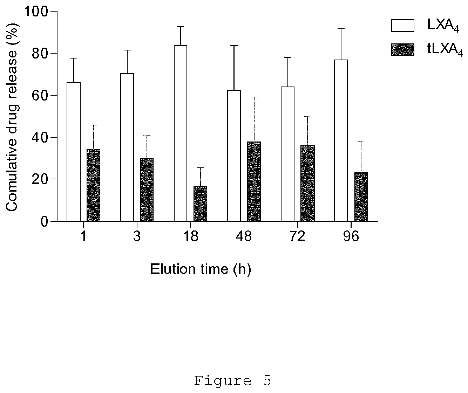

LXA₄ releasing from the PEO/PDLLA membranes was investigated by incubating membranes with a PBS solution at pH 7.4 for varying times. LXA₄ was quantitated by RP-HPLC. Figure 4 shows that approximately 15% of the total LXA₄, added to membranes, was released within 3 h. The maximum release was observed after 48 h (17%), followed by a progressive decrement at 72 h and 96 h (around 11%). Approximately 25% of LXA₄ was recovered as trans isomer, in line with the content of Romano M, Serhan CN. Lipoxin generation by permeabilized human platelets. Biochemistry. 1992;31(35):8269-77), as shown in figure 5.

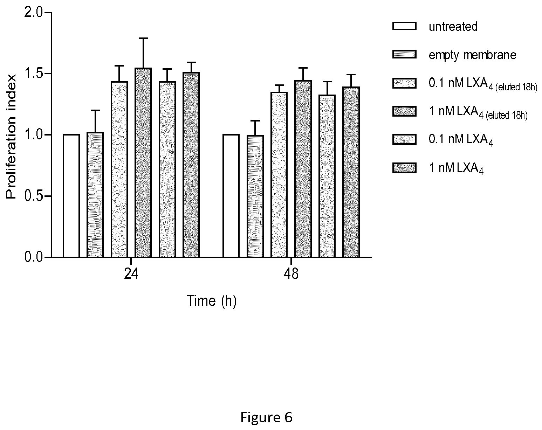

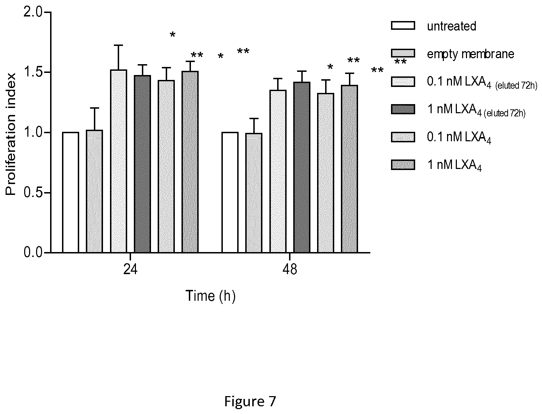

The proliferation and migration of hPDLSC growth a direct contact with fragment of LXA₄-loaded membrane were evaluated. 1 cm² sections of, not-loaded or LXA₄-loaded, membrane were added to adherent hPDLSCs, cultured in basal medium. Membranes were added on top of cellular monolayers, and not vice versa, to maximize the contact with the membrane. The LXA₄-loaded membranes stimulated proliferation, as shown in figure 6 and 7. The results demonstrate that LXA₄ can be loaded into membrane and released progressively in an aqueous environment without losing its biological activities.

The above experiments show that PEO/PDLLA bio-membranes can be loaded with LXA₄, acquiring pharmaceutical features.

The electrospinning method allows to produce large amounts of fibers with micrometer or nanometer dimensions in a short period of time, so to obtain a three-dimensional biodegradable fibrous membrane of PEO and PDLLA, consisting of random oriented fibers with homogeneous diameter of approximately 0.8 µm, as revealed by morphological characterization. The obtained membranes are highly porous, with average pore diameters of around 15 µm, which makes them ideal for incorporation within the fibers of biological active molecules, and for supporting and cell proliferation, thanks to easy diffusion of gases and nutrients. The membranes showed excellent capability to serve as scaffold, where hPDLSCs adhere and spread on the scaffold surface, making filopodia and long active cytoplasmic processes, indices of tight intercellular contacts and plasticity. The PEO/PDLLA membranes did not negatively affect cell proliferation and viability, indicating that these meshes are highly biocompatible.

LXA₄ was successfully incorporated into PEO/PDLLA membranes, from where it is released. LXA₄, after the releasing, retained its biological activities on hPDLSCs.

## Claims

1. Delivery system comprising a porous membrane consisting of fibers of polyoxyethylene (POE) and poly-DL-lactide (PDLLA) and LXA₄ wherein the fibers of polyoxyethylene (POE) and poly-DL-lactide (PDLLA) are random oriented and have a diameter comprised between 0,8 and 1,2 µm, in the in porous membrane the pore size in comprised between 5 and 15 µm, the porous membrane have a glass-transition temperature (Tg) comprised between 45 and 50 °C, the porous membrane have a melting peak comprised between 59 and 64 °C.

2. Delivery system according to claim 1 wherein the fibers of polyoxyethylene (POE) and poly-DL-lactide (PDLLA) have a diameter of 1µ.

3. Delivery system according to claim 1 wherein in the porous membrane the pore size is 15 µm.

4. Delivery system according to claim 1 wherein the porous membrane have a glass-transition temperature (Tg) of 47°C.

5. Delivery system according to anyone of the claims from 1 to 4 for use as a medicament.

6. Delivery system according to anyone of the claims from 1 to 4 for the treatment of periodontal diseases.

7. Use according to claim 5 wherein periodontal disease is gingivitis or periodontitis or pyorrhea.

8. Electrospinning process for the preparation of the delivery system of claim 1 comprising the following steps:
a) Preparation of a polymeric solution;
b) Loading the polymeric solution as prepared in step a) in a glass syringe with metallic needle;
c) Applying high voltage;
d) Extruding the solution from the needle at constant flow rate to form polymeric fibers which deposited onto a metallic collector up to the formation of a membrane;
Wherein in step a), for the preparation of the polymeric solution, polyoxyethylene (POE) and poly-DL-lactide (PDLLA) are separately dissolved in N,N-dimethylformamide (DMF) and then mixed and added of LXA₄, the amount of polyoxyethylene (POE) in the mixture is between 1 and 6 weight % of the total weight of N,N-dimethylformamide (DMF), the amount of poly-DL-lactide (PDLLA) in the mixture is between 2 and 12 weight % of the total weight of N,N-dimethylformamide (DMF),
in step c) the applied voltage is between 9 and 20 kV,
in step d) constant flow rate means that the flow rate is between 10 e 20 µl/min, the distance between the metallic needle and the metallic collector is between 10 and 30 cm.

9. Delivery system comprising a porous membrane consisting of fibers of polyoxyethylene (POE) and poly-DL-lactide (PDLLA) and LXA4 wherein the fibers of polyoxyethylene (POE) and poly-DL-lactide (PDLLA) are random oriented and have a diameter comprised between 0,8 and 1,2 µm, in the in porous membrane the pore size in comprised between 5 and 15 µm, the porous membrane have a glass-transition temperature (Tg) comprised between 45 and 50 °C, the porous membrane have a melting peak comprised between 59 and 64 °C obtained by an electrospinning process comprising the following steps:
a) Preparation of a polymeric solution;
b) Loading the polymeric solution as prepared in step a) in a glass syringe with metallic needle;
c) Applying high voltage;
d) Extruding the solution from the needle at constant flow rate to form polymeric fibers which deposited onto a metallic collector up to the formation of a membrane;
Wherein in step a), for the preparation of the polymeric solution, polyoxyethylene (POE) and poly-DL-lactide (PDLLA) are separately dissolved in N,N-dimethylformamide (DMF) and then mixed and added of LXA₄, the amount of polyoxyethylene (POE) in the mixture is between 1 and 6 weight % of the total weight of N,N-dimethylformamide (DMF), the amount of poly-DL-lactide (PDLLA) in the mixture is between 2 and 12 weight % of the total weight of N,N-dimethylformamide (DMF),
in step c) the applied voltage is between 9 and 20 kV,
in step d) constant flow rate means that the flow rate is between 10 e 20 µl/min, the distance between the metallic needle and the metallic collector is between 10 and 30 cm.
